# FASCICULE DE BREVET EUROPEEN

(11) **EP 0 512 926 B1**
(45) Date de publication et mention de la délivrance du brevet: **30.07.1997**
(21) Numéro de dépôt: 92401279.2
(22) Date de dépôt: 07.05.1992
(51) Int. Cl.: C08G 73/02, H01B 1/12, C07C 309/10, C07C 309/11

(54) **Polyanilines conductrices auto-dopées et leur procédé de préparation**
Leitfähige selbstdopierte Polyaniline und Verfahren zu ihrer Herstellung
Self-doped conductive polyanilines and process for their preparation

(30) Priorité: 07.05.1991 FR 9105578
(43) Date de publication de la demande: 11.11.1992
(73) Titulaire: ALCATEL N.V., NL-1077 XX Amsterdam (NL)
(72) Inventeur: Galaj, Stanislas, F-94110 Arceil (FR); Le Mehaute, Alain, F-91190 Gif Sur Yverte (FR)
(74) Mandataire: Laroche, Danièle

(56) Documents cités:
- EP-A- 0 128 272
- WO-A-89/01694
- FR-A- 2 152 889
- US-A- 4 940 517
- DATABASE WPIL Derwent Publications Ltd., London, GB; DATABASE WPIL, accession no. 88073879 & JP-A-63027467

## Description

La présente invention a pour objet des polyanilines conductrices auto-dopées, leur procédé de préparation et leur application dans le domaine de l'électromagnétisme. Elle se rapporte également à la préparation de l'une des matières premières employées pour l'obtention de ces polyanilines.

La polyaniline est un polymère dont les unités monomères correspondent à la formule: 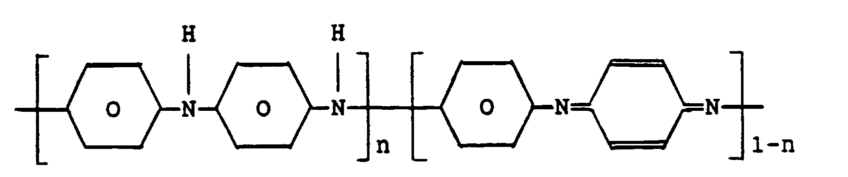 dans laquelle n est compris entre 0 et 1.

Deux types de noyaux sont présents dans la polyaniline: des noyaux benzéniques et quinoniques correspondant à des états d'oxydation différents représentés par la variable n. Pratiquement, on considère trois types de polyaniline: la forme réduite, demi-oxydée et oxydée. La forme réduite correspond à 100% de noyaux benzéniques (n = 1), la forme demi-oxydée à 75% de noyaux benzéniques et 25% de noyaux quinoniques (n = 0,5), la forme oxydée à 50% de noyaux benzéniques et 50% de noyaux quinoniques (n = 0). Néanmoins, la répartition et la périodicité des noyaux benzéniques et quinoniques le long de la chaîne est statistique. Le rapport d'oxydation est contrôlé par la quantité d'oxydant ajouté au milieu réactionnel de polymérisation.

Il se trouve que la forme demi-oxydée, appelée polyéméraldine, possédant autant de groupements amines (N-H) que de groupements imines (-N=), est celle qui devient la plus conductrice après fixation d'hydracide; cette opération est appelée "dopage".

La conduction ainsi obtenue dans le cas de la polyaniline correspond à un dopage par H⁺. Le polymère est donc conducteur lorsque le squelette est protoné. Ce dopage est réversible, mais peu stable vis-à-vis d'une éventuelle déprotonation, par exemple par lavage à l'eau ou par traitement thermique qui évapore l'hydracide.

Afin de stabiliser la conductivité de ces polymères, il a été proposé de les doper par greffage. Néanmoins, la présence d'un nombre trop élevé de chaînes latérales diminue la conductivité en réduisant les zones de contact entre chaînes. Ainsi se pose le problème de la régularité du positionnement des greffons, afin de ne pas avoir une densité linéique de greffons trop élevée à un endroit précis de la chaîne, ce qui nuit à la conduction.

Ce greffage a tout d'abord été effectué par action d'un dopant sur des polyanilines non-conductrices (brevet WO-8 901 694). Les sites amines ou imines, correspondant respectivement aux noyaux benzéniques et quinoniques, sont les sites affectés par les greffages connus. Ce greffage ne s'effectue donc que sur les atomes d'azote, et non sur les noyaux. Par exemple le brevet US-4 806 271 décrit un greffage sur l'atome d'azote de chaîne alkyle ou aryle portant une fonction terminale. Lorsque les sites immines de la chaîne sont greffés, ils ne peuvent plus fixer les protons, condition nécessaire à une bonne conductivité.

Des polyanilines conductrices présentant une stabilité accrue vis-à-vis de la déprotonation, peuvent être obtenues par "auto-dopage", c'est à dire l'introduction d'une fonction acide liée solidement à la chaîne.

Des polyanilines auto-dopées obtenues par alkylation de l'atome d'azote, par des chaînes portant une fonction acide sulfonique terminale ont été décrites (Synthetic Metals, 31 (1989) pp. 369-378). Il s'agit d'un traitement ultérieur effectué sur une polyaniline standard. Dans ce cas également le greffage est effectué sur l'atome d'azote.

Les efforts de recherche se sont donc dirigés vers l'obtention de polyanilines greffées sur les noyaux, ce qui ne peut être obtenu que par la polymérisation de monomères déjà porteurs d'une fonction acide sur le noyau.

Néanmoins, les polyanilines portant des greffons courts sur le noyau ne présentent aucune différence dans les caractéristiques physico-chimiques avec les polyanilines non dopées, en ce qui concerne en particulier la conductivité de la chaîne. Si la polyaniline est traitée par de l'oléum, une partie des noyaux sont greffés par -SO₃H, ce qui conduit à une faible augmentation de conductivité, jusqu'à un niveau voisin de 10⁻³ S.cm⁻¹ (Mol.Liq. Cryst. 189 (1990) 255).

Un polymère obtenu à partir d'un monomère ayant un greffon sur le noyau porteur d'une fonction donneur de protons (-COOH) a été décrit dans le brevet US-4 940 517, mais cette fonction est très insuffisante pour doper le polymère. De plus, le procédé d'homopolymérisation limite la longueur du greffon utilisable.

En effet, on sait que la polymérisation dans le milieu de polymérisation classique (HC1/(NH₄)₂S₂O₈ par exemple) de monomères portant un groupe d'une taille substantielle (au moins trois maillons) est très difficile du fait de l'encombrement stérique qui limite la polymérisation "tête-à-queue" ("head-to-tail"), sauf dans des milieux spécifiques par exemple HF-NH₄F au point eutectique, par voie électrochimique (Synthetic Metals, 29 (1989), pp. 1377, 1382).

Enfin, il n'est pas possible par les méthodes précédement décrites de faire varier de façon contrôlée la conductivité du polymère en fonction de la valeur que l'on souhaite obtenir.

Le problème technique que se propose de résoudre l'invention est de produire et de disposer d'une nouvelle classe de polyanilines conductrices, qui présentent des propriétés conductrices et une caractérisation améliorées par rapport à celles des polyanilines préparées jusqu'à présent, et notamment dont les paramètres de propagation d'impédance électromagnétique Z et la constante de propagation sont plus avantageuses que celles des polyanilines selon l'art antérieur. Lesdites polyanilines possèdent de plus l'avantage d'être très stables, au regard de leur éventuelle déprotonation, vis-à-vis de l'eau ou d'une élévation de température.

La présente invention a pour objet des polyanilines conductrices auto-dopées, caractérisées en ce les noyaux benzéniques et/ou quinoniques de ces polyanilines portent des greffons fonctionnalisés, ou substituants, correspondant à la formule:

-A-Z

dans laquelle:
A est un radical hydrocarboné de 2 à 8 atomes de carbone interrompu par au moins un hétéro-atome;
Z est un groupe fonctionnel choisi parmi les résidus d'un acide fort ou de l'un de ses sels.

De préférence, ledit groupe -A- est un radical hydrocarboné de 2 à 8 atomes de carbone interrompu par au moins un hétéro-atome choisi parmi O et S. Par exemple, l'atome d'oxygène exerce un effet donneur qui stabilise la charge positive de la chaîne principale.

Selon un premier mode de réalisation des polyanilines de l'invention, ledit groupe -A- est choisi dans le groupe des résidus ayant une fonction éther ou polyéther, tel que ceux correspondant à la formule: -O-(CH₂)ₙ-, -(CH₂O)ₙ- avec n supérieur ou égal à 2; -CH₂-O-(CH₂)ₙ-, -(CH₂-CH₂O)ₙ- avec n supérieur ou égal à 1; ou ayant une fonction ester, tels que -COO-(CH₂)ₙ-, -CH=CH-COO-(CH₂)ₙ- avec n supérieur ou égal à 1.

Selon un autre mode de réalisation des polyanilines de l'invention, ledit groupe -A- comprend, dans sa chaîne, un atome d'oxygène, et correspond à la formule:

-(CH₂)ᵣ-O-(CH₂)ₛ-

dans laquelle:
r est un entier valant 0 ou 1; et
s est un entier valant 3 ou 4.

Selon une autre forme d'exécution des polyanilines de l'invention, ledit groupe -Z est un groupe fonctionnel choisi parmi les résidus d'acide sulfonique, phosphonique et phosphorique, et de leurs sels.

La fonction acide sulfonique est la fonction préférée. Des sels tels que des sels métalliques, sont aussi envisagés. Les sels préférés sont les sels alcalins (Na, K, ...).

Selon encore une autre forme d'exécution des polyanilines de l'invention, leur squelette correspond à celui de la polyéméraldine.

Avantageusement, le nombre de greffons fonctionnalisés est égal à la moitié du nombre de noyaux benzéniques et/ou quinoniques, ce qui conduit à la conductivité maximale. Ceci peut être réalisé par l'ajustement des proportions réciproques de chacun des monomères.

Par exemple si la molécule de monomère A ne porte qu'un substituant, la conductivité maximale du polymère sera obtenue par une incorporation équimoléculaire du monomère A et du monomère B ; par contre, si le monomère A porte deux substituants, il ne faudra incorporer qu'une molécule du monomère A pour trois molécules du monomère B.

D'autre part, on peut faire varier leur conductivité en faisant varier le rapport moléculaire monomère A/monomère B. Par exemple, en diminuant la proportion du monomère A par rapport au monomère B, il est possible d'abaisser la conductivité car on diminue le nombre de greffons fonctionnalisés par rapport au nombre total de noyaux.

La présente invention a en outre pour objet un procédé de préparation de polyanilines conductrices auto-dopées, comprenant une étape de copolymérisation en milieu acide et en présence d'un oxydant d'un premier monomère fonctionnalisé A de formule: 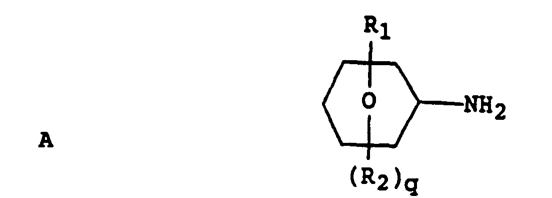 dans laquelle:
q vaut 0 ou 1,
R₁ et R₂, identiques ou différents, sont des groupes du type -A-Z dans lequel -A- est un radicale hydrocarboné comportant de 2 à 8 atomes de carbone, interrompu par au moins un hétéro-atome, et -Z est un groupe fonctionnel choisi parmi les résidus d'un acide fort ou de l'un de ses sels,
et d'un deuxième monomère non fonctionnalisé B de formule: 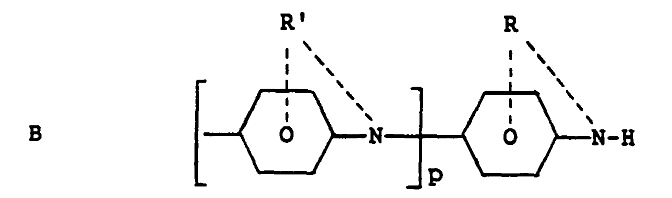 dans laquelle:
p vaut 0 ou 1,
R et R' identiques ou différents (la ligne en pointillés indiquant que R et/ou R' sont liés soit à l'atome d'azote, soit au noyau) sont choisis parmi:
   . -H, -OH, un radical alkyle contenant de 1 à 12 atomes de carbone, un groupe -CH₂OH, -C₂H₄OH, -COOH, -OCH₃, et -OC₂H₅, lorsqu'ils sont rattachés au noyau,
   . -H, -OH, un radical alkyle, un radical phényle, un groupe -CH₂OH, -C₂H₄OH, et -COOH, lorsqu'ils sont attachés à l'atome d'azote.

Les groupes R et R' sont choisis tels qu'ils permettent d'assurer, la compatibilité avec la matrice pour la réalisation de composites.

La copolymérisation comporte une phase d'induction durant laquelle l'oxydant est présent en faible quantité dans le milieu de copolymérisation et une phase de production.

Le terme "phase d'induction" correspond à la période d'initiation de la réaction, par exemple par voie thermique ou photochimique. Le terme "phase de production" correspond à la période de temps au cours de laquelle a lieu la croissance du polymère.

Selon une forme d'exécution du procédé de l'invention ledit monomère A seulement est présent en totalité dans le milieu réactionnel au début de la réaction et le monomère B et l'oxydant sont ajoutés par incréments ou en continu sur la période des phases d'induction et de production, le rapport monomère B/oxydant fixé étant sensiblement constant.

Selon une autre forme d'exécution du procédé, ledit monomère B est également présent dans le milieu de copolymérisation au cours de la phase d'induction.

Selon encore une autre forme d'exécution du procédé lesdits monomères A et B sont présents en totalité dans le milieu réactionnel au début de la réaction, et l'oxydant est ajouté par incréments ou en continu sur la période des phases d'induction et de production.

Selon une autre forme d'exécution du procédé selon l'invention, le milieu réactionnel est soumis au cours de la phase d'induction à un chauffage conduisant à une température pouvant atteindre 40°C pendant quelques minutes, puis on laisse la température du milieu réactionnel évoluer de façon inertielle.

Le milieu de polymérisation est n'importe quel solvant inerte vis-à-vis des réactifs, par exemple de l'eau. Par "milieu acide", on entend que le pH est inférieur à 1. Comme acides appropriés, on peut citer les acides chlorhydrique, sulfurique, phosphorique et autres, et leurs mélanges, ainsi que l'eutectique HF-NH₄F. L'acide préféré est l'acide sulfurique. Le terme "milieu oxydant" signifie que le milieu contient un oxydant qui peut être KIO₃, H₂O₂, (NH₄)₂S₂O₈, (NH₄)₂Cr₂O₇, et autres. L'oxydant employé de préférence consiste en (NH₄)₂S₂O₈ ou KIO₃.

Ledit monomère B peut être de l'aniline, ou le dimère, la N-phényl-p-phénylènediamine, non substituée ou substituée sur le noyau ou l'azote par un groupe choisi parmi -OH, -CH₂OH, -C₂H₄OH, -COOH, alkyl, -OCH₃, -OC₂H₅. Le monomère B préféré est l'aniline. Des mélanges de monomères B peuvent être envisagés.

Ledit monomère A est de l'aniline mono- ou disubstituée sur le noyau, en position ortho ou méta-. Ainsi, ce monomère A est du type (o)R₁-aniline, (m)R₁-aniline, (o)R₁,(o)R₂₋aniline ou (o)R₁,(m)R₂₋aniline, m(R₁),(o)R₂₋aniline, (m)R₁,(m)R₂₋aniline. Des mélanges de monomères A peuvent également être envisagés.

Comme exemple de groupe R₁ (et/ou R₂), on peut citer:-CH=CH-COO-(CH₂)₃SO₃H; -CH=CH-COO-(CH₂)₄SO₃H; -COO(CH₂)₃SO₃H; -COO(CH₂)₄SO₃H; -O(CH₂)₃SO₃H; -O(CH₂)₄SO₃H; -CH₂O(CH₂)₃SO₃H; -CH₂O(CH₂)₄SO₃H; et leurs sels de sodium, potassium, ou ammonium.

Le choix du groupe R₂ doit permettre d'assurer la compatibilité avec la matrice pour la réalisation de composites.

De préférence, ledit monomère A est de l'aniline disubstituée en ortho.

En effet, au cours de l'opération de polymérisation, l'association de deux monomères peut conduire à la formation de benzidine cancérigène: 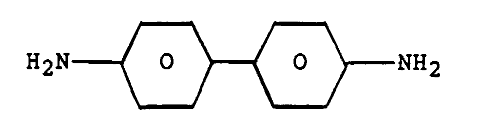 ceci ne se produit plus dès lors que chacun des monomères porte deux substituants en position ortho, pour des raisons d'encombrement stérique.

De préférence, ledit monomère A porte une fonction acide sulfonique. L'introduction d'une fonction acide sulfonique sur le noyau d'une arylamine supprime dans la plupart des cas l'activité génotoxique.

La présente invention a aussi pour objet les polyanilines obtenues selon le procédé précédemment décrit.

Selon un mode de réalisation préféré, les polyanilines sont obtenues à partir d'aniline, en tant que monomère B, et d'aniline substituée en ortho ou méta par un groupe de formule:

-(CH₂)ᵣ-O-(CH₂)ₛ-SO₃H

dans laquelle:
r vaut 0 ou 1;
s vaut 3 ou 4;
ou le sel alcalin correspondant.

Les polyanilines particulièrement préférées sont obtenues à partir d'aniline en tant que monomère B et en tant que monomère A, soit l'acide 3-(3-aminobenzyloxy)-1-propanesulfonique, soit l'acide 4-(2-aminophénoxy)-1-butanesulfonique, ou leurs sels de sodium.

La présente invention se rapporte aussi au procédé de préparation des monomères de type A, utilisés pour l'obtention desdites polyanilines, qui consiste, à partir d'un dérivé nitré aromatique portant au moins un groupement hydroxyle ou hydroxylé en position ortho ou méta:
. à réaliser le dérivé métallique de ce dérivé (alcoolate, phénolate),
. à faire réagir ce dernier sur une sultone en milieu anhydre, polaire et aprotique,
. à réduire la fonction "nitro" en fonction "amino",
. à récupérer le produit final sous forme de sel interne ou d'un dérivé salin quelconque en fonction de la commodité de séparation;
suivant le schéma réactionnel:

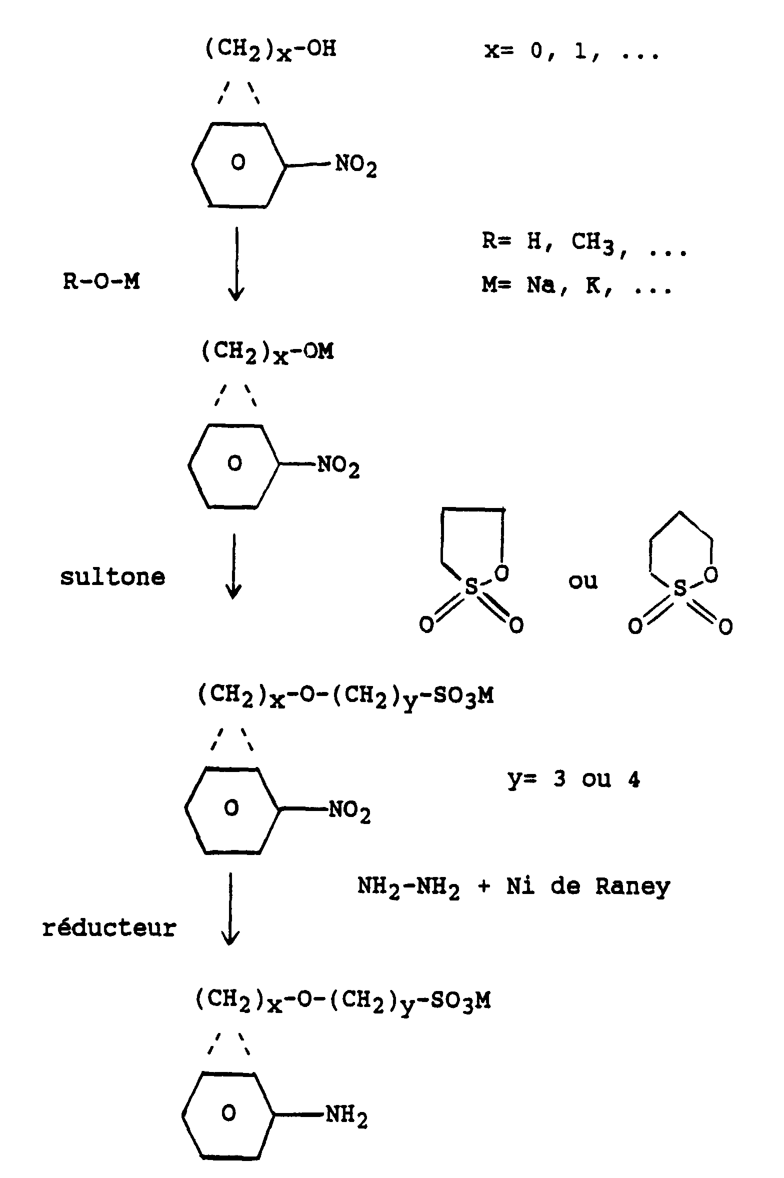

Les lignes en pointillés indiquent que le substituant peut se trouver en position ortho ou méta.

Chacune de ces formes peut être directement utilisée pour la polymérisation en milieu acide en présence d'oxydant.

Ainsi, par exemple, le dérivé nitré et hydroxylé peut être choisi parmi: l'orthonitrophénol, le métanitrophénol, l'orthonitrobenzylalcool, le métanitrobenzylalcool, le 2-nitrorésorcinol, l'acide 3-nitrocinnamique, et autres. Ce dérivé, en présence de NAOH ou CH₃ONa, conduit au sel métallique qui réagit avec une sultone, celle-ci peut être choisie parmi la 1,3-propanesultone ou la 1,4-butanesultone. Le monomère A est alors obtenu par réduction, par exemple par l'hydrazine, en présence de nickel de Raney comme catalyseur.

Suivant un autre mode de réalisation, lesdits monomères de type A sont préparés à partir d'un dérivé aminé portant au moins un groupement hydroxyle ou hydroxylé en position ortho ou méta, selon un procédé qui consiste:
. à réaliser le dérivé métallique de ce dérivé (alcoolate, phénolate),
. à faire réagir ce dernier sur une sultone en milieu anhydre, polaire et aprotique,
. à récupérer le produit final sous forme de sel interne ou d'un dérivé salin quelconque en fonction de la commodité de séparation; suivant le schéma réactionnel:

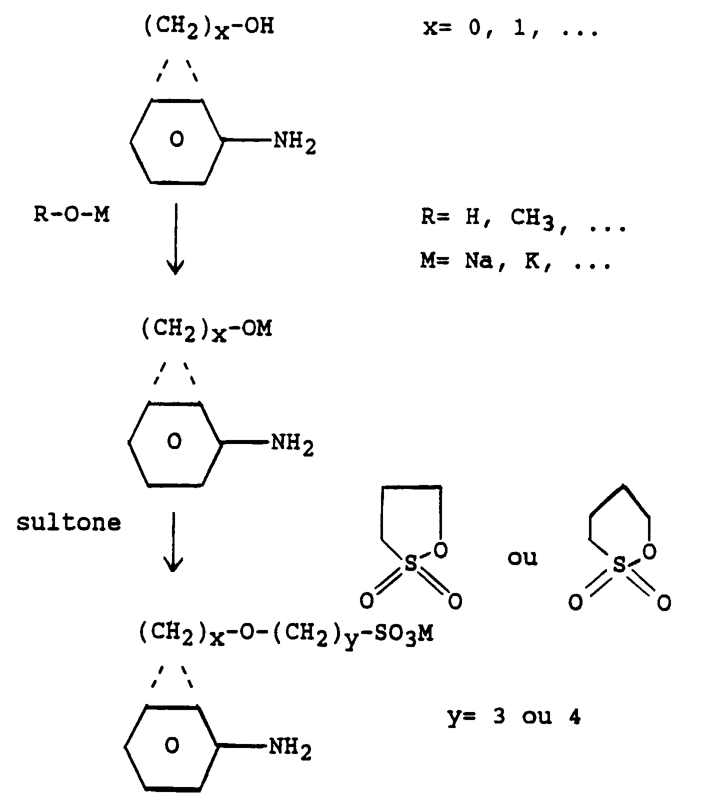

Les lignes en pointillés indiquent que le substituant peut se trouver en position ortho ou méta.

Chacune de ces formes peut être directement utilisée pour la polymérisation en milieu acide en présence d'oxydant.

Ainsi, par exemple, le dérivé aminé et hydroxylé peut être choisi parmi: l'orthoaminonitrophénol, le métaaminophénol, l'orthoaminobenzylalcool, le métaaminobenzylalcool, le 2-aminorésorcinol, l'acide 3-aminocinnamique, et autres.

Les polyanilines conductrices auto-dopées obtenues par ce procédé sont mises en solution dans un solvant. Ledit solvant peut être une solution aqueuse basique telle que la soude ou la potasse, une solution basique organique, et de préférence une base faible volatile telle que l'ammoniaque, la monométhylamine, la diméthylamine, la triméthylamine, la monoéthylamine, la diéthylamine, la triéthylamine, la pyrrolidine, la pyridine. De préférence, les polyanilines auto-dopées obtenues par ce procédé sont mises en solution dans l'ammoniaque. La solution est mise en oeuvre dans un procédé de revêtement par peinture, pistolage, ou trempage. Un simple traitement thermique permet de régénérer le polymère auto-dopé, par exemple sous forme de couches minces ou de dépôts sur une surface plane ou dans un poreux.

La présente invention concerne également les applications électromagnétiques des polyanilines précitées. Ces polyanilines peuvent être utilisées dans un grand nombre de domaine, comme le domaine des écrans semi-conducteurs ou du blindage électromagnétique, ou le domaine des hyperfréquences.

D'autres caractéristiques et avantages de la présente invention apparaîtront au cours de la description suivante de modes de réalisation donnés à titre illustratif mais nullement limitatif, et en regard des dessins annexés où:
- les figures 1 et 2 donnent les paramètres Zγ et γ/Z en fonction du logarithme de la fréquence d'analyse pour un polymère selon la présente invention;
- la figure 3, relative à la constante diélectrique, donne le diagramme ε'/ε" pour le même polymère;
- la figure 4 représente les points expérimentaux lors de la mesure de la conductivité a sous basse tension pour un second polymère selon la présente invention;
- les figures 5 et 6 donnent des résultats des tests d'analyse thermique différentielle/analyse thermogravimétrique sous atmosphère d'azote et d'argon respectivement pour un troisième polymère selon la présente invention, tel que défini dans l'exemple 13 ci-dessous.

### EXEMPLE 1 - Préparation du monomère A1

Dans un erlenmeyer surmonté d'un réfrigérant et d'un piège à vapeur d'eau, on introduit 15,3g d'alcool 3-nitrobenzylique (0,1 mole), 100ml de toluène et 12g de carbonate de sodium anhydre en poudre fine. La suspension est agitée et chauffée à reflux pendant 24 heures. On ajoute 13,5g de 1,3-propanesultone, et l'on maintien le chauffage à reflux pendant 8 heures.

La suspension est évaporée sous vide, et le résidu dissous dans 15Oml d'eau. On ajoute à cette solution 2Og de fer en poudre et 10ml d'acide chlorhydrique concentré. La suspension est agitée et chauffée vers 80°C environ pendant 1 heure, puis filtrée. Le filtrat est additionné de soude, et filtré à nouveau pour éliminer l'abondant précipité d'hydroxydes de fer. Le filtrat est acidifié par HCl, puis évaporé sous vide. Le résidu est repris à chaud par de l'éthanol anhydre, puis la suspension est filtrée pour éliminer NaC1. L'évaporation du filtrat sous vide conduit à un résidu visqueux cristallisant partiellement après quelques jours. Les cristaux sont essorés et recristallisés dans du méthanol.

La poudre rose clair obtenue a une masse de 8,3g. Le rendement global est de 34%.

On obtient ainsi l'acide 3-(3-aminobenzyloxy)-1-propanesulfonique présentant la formule développée: 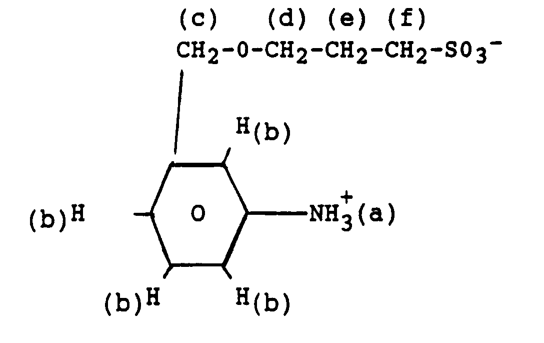 dont le spectre RMN (60 MHz dans le DMSO-d₆) est le suivant:
- a:: bande large entre 8,7 et 10,6 ppm
- b :: 7,5 ppm (triplet)
7,3 ppm (multiplet)
- c:: 4,5 ppm (singulet)
- d:: 3,5 ppm (triplet)
- e:: 1,9 ppm (multiplet)
- f:: 2,5 ppm (multiplet)

### EXEMPLE 2 - Préparation du monomère A2

30,9g de 2-nitrophénol à 10% d'humidité (0,2 mole) sont dissous à chaud dans 150ml d'eau contenant 8,2g de soude. Cette solution est évaporée sous vide avec montée progressive de la température jusqu'à 110°C. Les cristaux rouges anhydres obtenus sont dissous dans 150ml de diméthylsulfoxyde anhydre contenant 31,3g de 1,4-butanesultone (0,23 mole). Cette solution, en flacon étanche, est placée en étuve à 90°C pendant un temps pouvant aller jusqu'à 48 heures. La solution est alors concentrée sous vide puis, par addition de 1-propanol et refroidissement, des cristaux de 4-(2-nitrophénoxy) - 1-butanesulfonate de sodium précipitent.

Les cristaux sont essorés puis dissous dans un mélange constitué de 200ml d'eau et 100ml d'éthanol. Cette solution est chauffée vers 60-70°C. On y ajoute 30ml d'hydrate d'hydrazine, et environ 1 à 2g de nickel de Raney en poudre: la réduction de la fonction nitro commence. Deux autres ajouts d'environ 1g de nickel de Raney sont effectués à 30 minutes d'intervalle.

La solution est alors portée à ébullition pendant 20 minutes. Après refroidissement, la suspension est filtrée et le filtrat évaporé sous vide. Le résidu obtenu est redissous à chaud dans un mélange 1-propanol/eau (95/5 en volume). Après refroidissement, des cristaux précipitent. Leur séchage à 60°C sous vide pendant 24 heures donne une poudre rose pâle de masse 41,4g. Le rendement global est de 70%.

L'analyse montre qu'il s'agit de 4-(2-aminophénoxy)-1-butane-sulfonate de sodium hydraté à 1,5 molécules d'eau. Ce sel peut être utilisé directement pour l'obtention de polyanilines "auto-dopées".

Le monomère A₂ a pour formule: 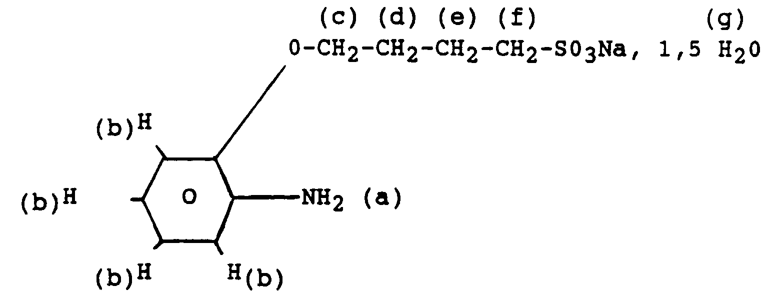 et son spectre RMN (60 MHz dans le DMSO-d₆) est le suivant:
- a:: 4,6 ppm (multiplet)
- b:: 6,6 ppm (multiplet)
- c:: 3,9 ppm (multiplet)
- d et e:: 1,7 ppm (multiplet)
- f:: 2,4 ppm (multiplet)
- g:: 3,3 ppm (singulet).
Autre mode de réalisation du monomère A2 en quantité plus importante:

309g de 2-nitrophénol à 10% d'humidité (2 moles) sont dissous à chaud dans un erlenmeyer contenant 1 litre d'eau et 82g de soude en pastilles. Cette solution est évaporée sous vide en montant progressivement la température jusqu'à 95°C. Aux cristaux rouges anhydres obtenus on ajoute 700ml de diméthylsulfoxyde anhydre et 313g de 1,4-butanesultone. La mixture est progressivement chauffée jusqu'à 90°C. La température est maintenue à cette valeur durant 24 heures, puis la solution de couleur orange est concentrée sous vide par distillation d'une grande partie du DMSO. Au résidu verdâtre obtenu, on ajoute 1 litre d'eau, 12g de soude et 50ml d'hydrate d'hydrazine. La solution est portée puis maintenue au voisinage de 70°C. On y ajoute environ 2g de nickel de Raney et la réduction commence. Des ajouts de 50ml d'hydrate d'hydrazine et d'environ 2g de nickel de Raney sont encore effectués 6 fois toutes les 30 minutes. Une demi-heure après le dernier ajout, la suspension est portée à l'ébullition pendant 2 heures, refroidie et filtrée. Le résidu d'évaporation sous vide du filtrat est redissous à chaud dans 2 litres de mélange 1-propanol/eau (95/5 en volume). La solution est laissée au repos pendant 3 jours aux environs de 10°C. Les cristaux obtenus sont essorés, rincés à l'éthanol anhydre et séchés sous vide à 50°C pendant 3 jours. La masse de poudre obtenue est de 434,5g. Le rendement global est de 74%.

### EXEMPLE 3 - Préparation du monomère A3

309g de 2-nitrophénol à 10% d'humidité (2 moles) sont dissous à chaud dans un erlenmeyer contenant 1 litre d'eau et 82g de soude en pastille. Cette solution est évaporée sous vide en montant progressivement la température jusqu'à 95°C. Aux cristaux rouges anhydres obtenus on ajoute 350ml de diméthylsulfoxyde anhydre et 270g de 1,3-propanesultone et l'on fixe un piège à vapeur d'eau sur l'erlenmeyer. La mixture est progressivement chauffée jusqu'à 90°C. La température est maintenue à cette valeur durant 24 heures, puis la solution de couleur orange est concentrée sous vide par distillation d'une grande partie du DMSO. Le résidu jaunâtre est redissous à chaud dans un mélange constitué de 1,5 litre d'éthanol, 1 litre d'eau et 8g de soude. La solution est portée, puis maintenue au voisinage de 70°C. On y ajoute 50ml d'hydrate d'hydrazine et environ 2g de nickel de Raney et la réduction commence. Des ajouts de 50ml d'hydrate d'hydrazine et d'environ 2g de nickel de Raney sont encore effectués 7 fois toutes les 30 minutes. La réduction est pratiquement terminée lorsque la mousse de couleur jaune devient blanche. Une demi-heure après le dernier ajout, la suspension est portée à l'ébullition pendant 1 heure, puis filtrée à chaud. Après refroidissement du filtrat, des cristaux blancs se déposent. Ils sont essorés, rincés à l'éthanol puis mis à sécher sous vide à température ambiante d'abord, puis à 50°C pendant 3 jours. Les liqueurs mères sont concentrées à chaud et sous vide de façon à récupérer le solde de produit qui sera traité comme le premier lot. La masse de poudre obtenue est de 200,8g pour le premier lot et de 264,5g pour le second (moins pur). Le rendement global de cette synthèse est de 83%. Le spectre RMN (60 MHz dans le DMSO-d₆) montre qu'il s'agit de 3-(2-aminophénoxy)-1-propanesulfonate de sodium hydraté à 1,5 molécules d'eau: 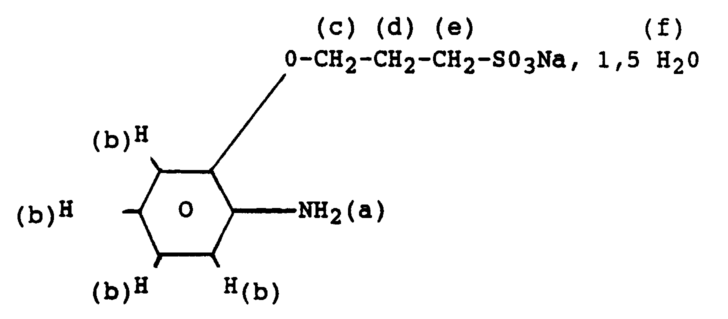 et son spectre RMN (60 MHz dans le DMSO-d₆) est le suivant:
- a:: 4,6 ppm (singulet)
- b:: 6,6 ppm (multiplet)
- c:: 4,0 ppm (triplet)
- d:: 2,0 ppm (multiplet)
- e:: 2,6 ppm (triplet)
- f:: 3,4 ppm (singulet)

### EXEMPLE 4 - Préparation du monomère A4

50g de 3-nitrophénol (environ 0,36 mole) sont dissous à chaud dans 200ml d'eau contenant 14,75g de soude. Cette solution est évaporée sous vide en montant progressivement la température jusqu'à 100°C. Les cristaux rouges anhydres obtenus sont dissous dans 70ml de diméthylsulfoxyde anhydre contenant 54g de 1,4-butanesultone. Cette solution mise en flacon étanche, est placée en étuve à 90°C pendant 48 heures. Le diméthylsulfoxyde est évaporé sous vide. Le résidu jaune pâle est redissous à chaud dans un mélange constitué de 300ml d'éthanol, 180ml d'eau et 1,5g de soude. La solution est portée au voisinage de 70°C. On y ajoute 75ml d'hydrate d'hydrazine et environ 2g de nickel de Raney, et la réduction commence. Elle est pratiquement terminée au bout de 15 minutes lorsque la mousse de couleur jaune devient blanche. La suspension est portée à l'ébullition pendant 1 heure, puis filtrée à chaud et sous vide, 600ml d'éthanol sont ajoutés au filtrat. Après refroidissement du filtrat, des cristaux blancs se déposent. Ils sont essorés, rincés à l'éthanol puis mis à sécher sous vide à température ambiante d'abord, puis à 50°C pendant 2 jours. La masse de poudre obtenue est de 72,3g. Le rendement global est est de 75%.

Le spectre RMN (60MHz dans le DMSO-d₆) montre qu'il s'agit de 4-(3-aminophénoxy)-1-butanesulfonate de sodium anhydre: 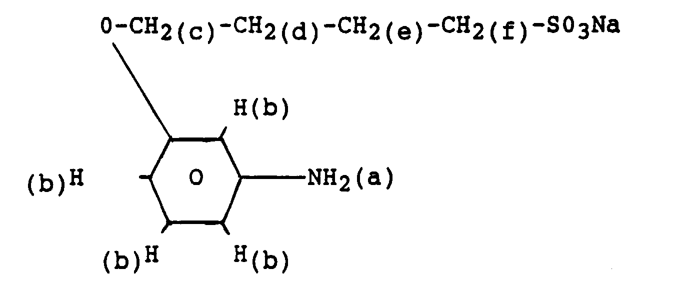 et son spectre est le suivant:
- a :: 5 ppm (singulet)
- b :: 6,1 ppm (multiplet)
6,9 ppm (triplet)
- c :: 3,8 ppm (multiplet)
- d et e:: 1,7 ppm (multiplet)
- f :: 2,4 ppm (triplet)

### EXEMPLE 5 - Préparation du monomère A5

50g de 3-nitrophénol (environ 0,36 mole) sont dissous à chaud dans 200ml d'eau contenant 14,75g de soude. Cette solution est évaporée sous vide en montant progressivement la température jusqu'à 100°C. Les cristaux rouges anhydres obtenus sont dissous dans 70 ml de diméthylsulfoxyde anhydre contenant 48,4g de 1,3-propanesultone. Cette solution mise en flacon étanche est placée en étuve à 90°C pendant 48 heures. Le diméthylsulfoxyde est évaporé sous vide. Le résidu jaune pâle est redissous à chaud dans un mélange constitué de 300ml d'éthanol, 180ml d'eau et 1,5g de soude. La solution est portée au voisinage de 70°C. On y ajoute environ 2g de nickel de Raney; 75ml d'hydrate d'hydrazine sont alors ajoutés goutte à goutte et la réduction commence. La réduction est pratiquement terminée au bout de 30 minutes lorsque la mousse de couleur jaune devient blanche. La suspension est portée à l'ébullition pendant 1 heure, puis filtrée sous vide après refroidissement. Le filtrat est évaporé et le résidu repris à chaud par 600ml d'un mélange éthanol/eau (95/5). Après refroidissement du filtrat, des cristaux blancs se déposent. Ils sont essorés, rincés à l'éthanol puis mis à sécher sous vide à température ambiante d'abord, puis à 50°C pendant 2 jours. La masse obtenue est de 54g.

On obtient ainsi le sel de sodium de l'acide 3-(3-aminophénoxy)-1-propanesulfonique.

### EXEMPLE 6 - Préparation du monomère A6

L'exemple 2 est répété mais en partant de 0,2 mole de 2-nitrorésorcinol, 16,4g de soude, et 62,6g (0,46 mole) de 1-butanesulfonate de sodium. On obtient le sel de sodium du 1-amino-2,6-bis(4-sulfobutoxy)benzène.

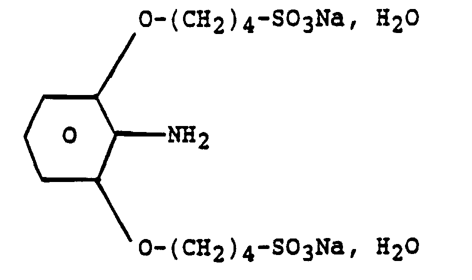

### EXEMPLE 7 - Copolymérisation des monomères A1 et B

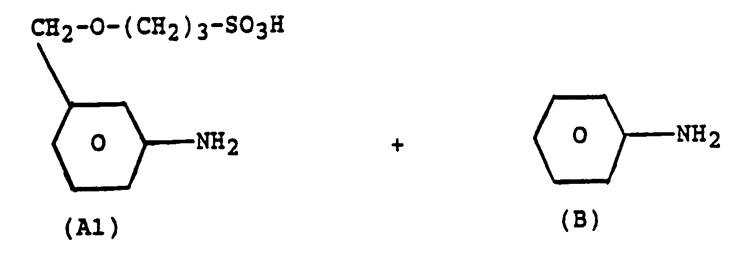

- A1 :: acide 3-(3-aminobenzyloxy)-1-propanesulfonique
- B :: aniline

### Mode opératoire

Un mélange constitué de 1,30g de monomère A1, 0,5g de monomère B, 10ml d'eau et 2,5ml d'acide sulfurique à 96% est agité à température ambiante. On y ajoute alors, par petites pincées, en 30 minutes environ, 2,5g de peroxodisulfate d'ammonium en poudre. La température monte d'environ 10 à 15°C. L'agitation se poursuit 2 heures. La suspension obtenue est filtrée et lavée à l'eau plusieurs fois jusqu'à ce que le filtrat soit à un pH d'environ 7. La masse noirâtre et collante obtenue est séchée sous vide 24 heures à 60°C. Le copolymère obtenu se présente sous la forme d'une fine poudre vert foncé de masse 0,955g.

A1 et B ont été employés en excès d'environ 20% par rapport à la quantité stoechiométrique d'oxydant nécessaire pour obtenir un copolymère dont le squelette soit celui de la polyéméraldine. Le calcul du rendement, effectué en se référant à l'oxydant, est de 65%

### EXEMPLE 8 - (comparatif)

L'homopolymérisation de A1, effectuée dans les mêmes conditions que celles de la copolymérisation A1 + B décrites dans l'exemple 7, conduit à une poudre de couleur brun-vert foncé avec un rendement, calculé par rapport à l'oxydant, de 30%.

### EXEMPLE 9 - (comparatif)

L'aniline seule polymérise en polyéméraldine avec des rendements de l'ordre de 80%.

### EXEMPLE 1O - Copolymérisation des monomères A2 et B

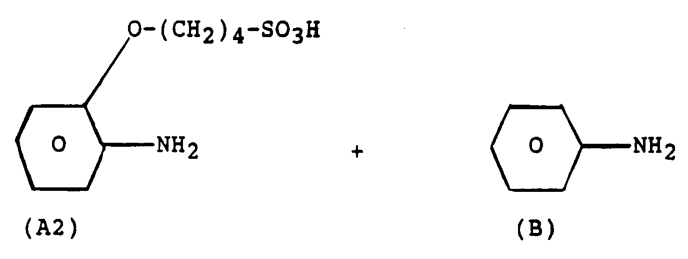

- A2 :: acide 4-(2-aminophénoxy)-1-butanesulfonique
- B :: aniline

### Mode opératoire

Un mélange constitué de 5,35g de monomère A2 sous forme de sel de sodium hydraté, 80ml d'eau et 20ml d'acide sulfurique à 96% est agité à température ambiante. On y ajoute, toutes les 10 minutes et en 8 fois, 0,2ml de monomère B et 1g de peroxodisulfate d'ammonium. Un neuvième ajout correspond au solde des réactifs: 1,7ml de monomère B et 9,7g de (NH₄)₂S₂O₈ ont été ainsi introduits. A2 et B sont en excès de 10% par rapport à la quantité stoechiométrique d'oxydant nécessaire pour obtenir un copolymère dont le squelette soit celui de la polyéméraldine.

Il est nécessaire de chauffer un peu (environ 30° à 40°C) pour accélérer le processus de copolymérisation car, après le deuxième ajout, rien ne se produit comme le montre la très faible variation de couleur du milieu réactionnel. L'agitation est poursuivie 1 heure après le dernier ajout. La suspension vert sombre, presque noire est très difficilement filtrable car assez visqueuse. Il est préférable de la diluer dans 500ml d'eau puis d'effectuer une centrifugation. Le culot est redispersé dans 200ml d'eau puis une nouvelle centrifugation est opérée. Cette opération est répétée encore une fois. Le culot constitué de copolymère humide est pratiquement débarrassé de tous ions étrangers. Un séchage de 24 heures sous vide à 60°C donne une poudre verte foncée, presque noire, de masse 2,35g.

Le rendement calculé en se référant à la quantité d'oxydant est de 41%.

Le polymère obtenu par copolymérisation de l'acide 4-(2-aminophénoxy)-1-butanesulfonique (A2) avec l'aniline est partiellement soluble dans l'eau en milieu neutre et l'ajout d'acide en reprécipite une partie. Dans ces deux cas les solutions sont vertes. La solubilité est totale en milieu basique (hydroxyde d'ammonium ou de sodium). Les solutions ainsi obtenues sont violettes. L'évaporation à chaud de la solution ammoniacale fournit des films minces et conducteurs de couleur verte.

Bien que dans la chaîne les fonctions imines et amines soient moins basiques que l'ammoniaque, un simple traitement thermique déplace l'équilibre et permet la régénération de la polyéméraldine auto-dopée.

Par contre, l'évaporation de la solution sodique ne permet pas la régénération du polymère conducteur comme le montre la couleur violette du film obtenu.

### EXEMPLE 11

Dans un erlenmeyer de 2 litres contenant un mélange de 150g de monomère A2 sous forme de sel de sodium hydraté, 49g d'aniline, 90ml d'acide sulfurique à 96% et 820ml d'eau, on ajoute, goutte à goutte pendant 15 heures, une solution composée de 100g de bichromate d'ammonium, 60ml d'acide sulfurique à 96% et 540ml d'eau. Le milieu réactionnel est maintenu aux environs de 25°C. La suspension visqueuse est centrifugée. Le culot est redispersé dans 2 litres d'eau additionnée de 50ml d'acide acétique puis centrifugé. On obtient un copolymère de couleur "vert foncé". Cette opération est répétée encore 3 fois puis le culot est mis à sécher sous vide primaire à 60°C. La masse de poudre obtenue est de 83,9g. Le rendement calculé par rapport à l'oxydant est de 64%

### EXEMPLE 12 - Copolymérisation des monomères A6 et B

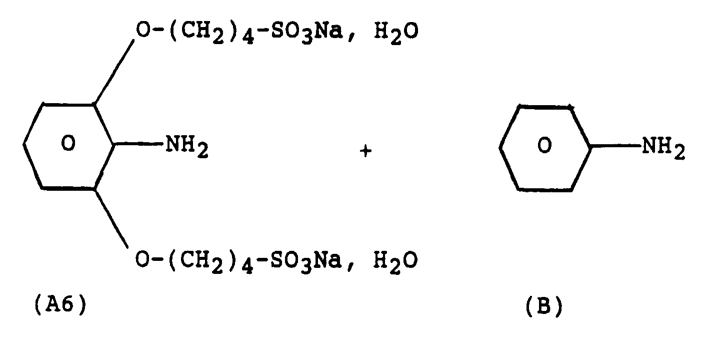

- A6 :: 1-amino-2,6-bis(4-sulfobutoxy)benzène
- B :: aniline

### Mode opératoire:

L'exemple 10 est répété mais en partant de 4,5g du monomère A6 et de 2,5ml d'aniline.

### EXEMPLE 13

La caractérisation hyperfréquence du polymère auto-dopé/greffé de l'exemple 10 ci-dessus est effectuée de la manière suivante:

On charge, dans un malaxeur, un mélange de polymère isolant constitué de 80% de polyéthylène et de 20% d'EPDM (élastomère), avec 25% en volume de polymère greffé décrit dans l'exemple 10.

On réalise avec ces matériaux une pastille de 7mm de diamètre et de 5mm de hauteur, percée d'un trou central. Cette pastille est positionnée dans un guide d'onde coaxial, lui-même branché aux bornes d'un analyseur de réseau de type Wiltron 360. Cet analyseur permet d'obtenir les paramètres de la propagation d'onde dans l'échantillon considéré comme un quadripôle. L'analyse est effectuée entre 40 MHz et 18 GHz et l'on tire par calcul les paramètres de propagation d'impédance électromagnétique Z de l'échantillon et la constante de propagation γ dans l'échantillon.

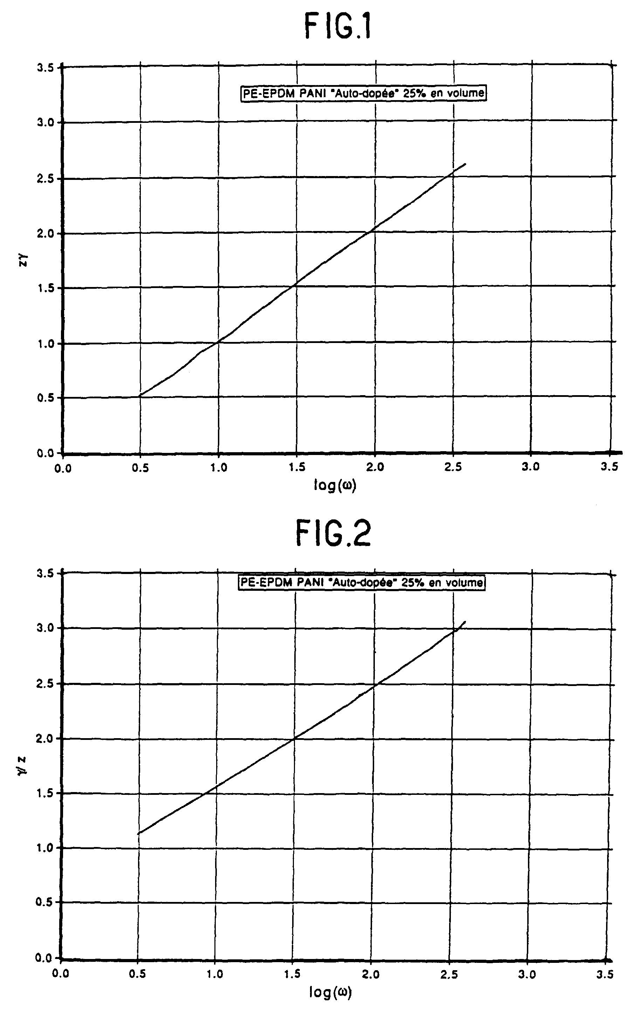

Les figures 1 et 2 donnent respectivement les paramètres Zγ et γ/Z en fonction du logarithme de la fréquence d'analyse. Nous voyons qu'il s'agit de droites sur le domaine d'analyse.

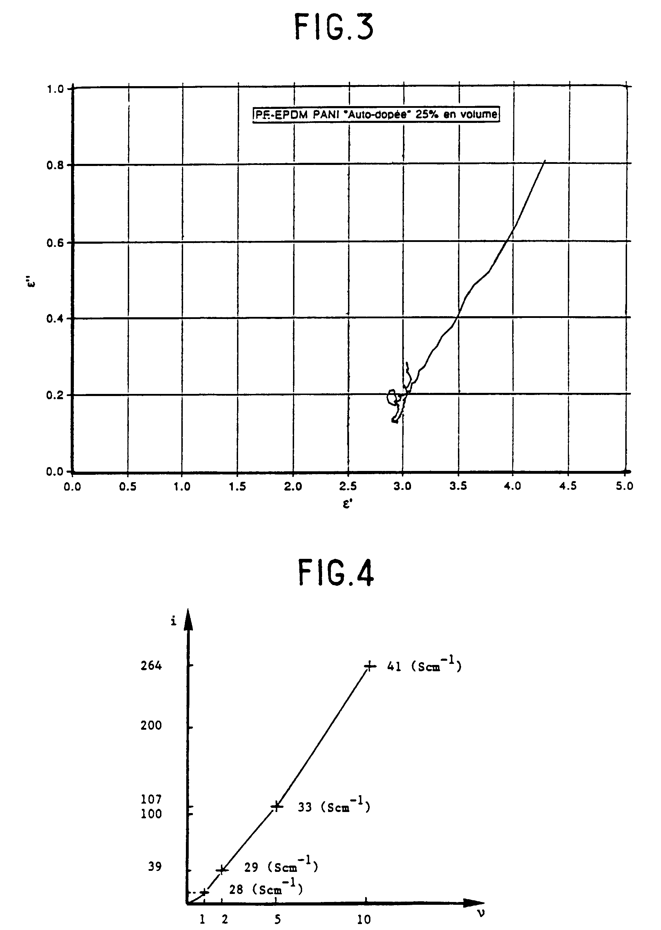

La figure 3, relative à la constante diélectrique, donne le diagramme complexe ε'/ε" qui fait apparaître une droite de régression de pente voisine de 0,4.

### EXEMPLE 14

Un erlenmeyer de 250 cc contient 1,7g d'aniline, 5g d'acide 4-(2-aminophénoxy)-1-butanesulfonique sous forme de sel de sodium hydraté. Le tout est en solution dans 55ml d'eau et 20ml d'acide sulfurique à 96%. On ajoute goutte à goutte 9,7g de peroxodisulfate d'ammonium en solution dans 25ml d'eau, de sorte que la durée d'ajout dure trois heures, et que la normalité du milieu passe de 9N en début de réaction à 7N en fin de réaction. Le temps d'agitation complémentaire est choisi égal à 1 heure. Après centrifugation et lavage, on obtient le polymère "vert bouteille" avec un rendement de 16%.

Puis on effectue sur ce polymère plusieurs types d'essais de caractérisation:
(1) Le produit est pastillé sans additif puis, au moyen d'une méthode de mesure de conductivité de type 4 pointes, on établit la valeur de la conductivité.
   On trouve dans ce cas que celle-ci σ est comprise entre 30 et 40 S.cm⁻¹, ce qui correspond, pour ce type de polymère conducteurs, à une excellente conductivité (figure 4).
(2) On effectue un diagramme de Debye afin d'établir l'état cristallographique du matériau. Ce diagramme montre qu'une partie du matériau est cristallisé, on observe en effet 2 anneaux de diffraction à 4,25 Angströms et 3,59 Angströms.

### EXEMPLE 15

L'exemple 14 est répété mais en changeant la normalité de début et de fin de réaction, respectivement 8N et 5N, obtenue en mettant 10ml d'acide sulfurique dans 30ml d'eau. Le rendement de copolymérisation est cette fois de 26%.

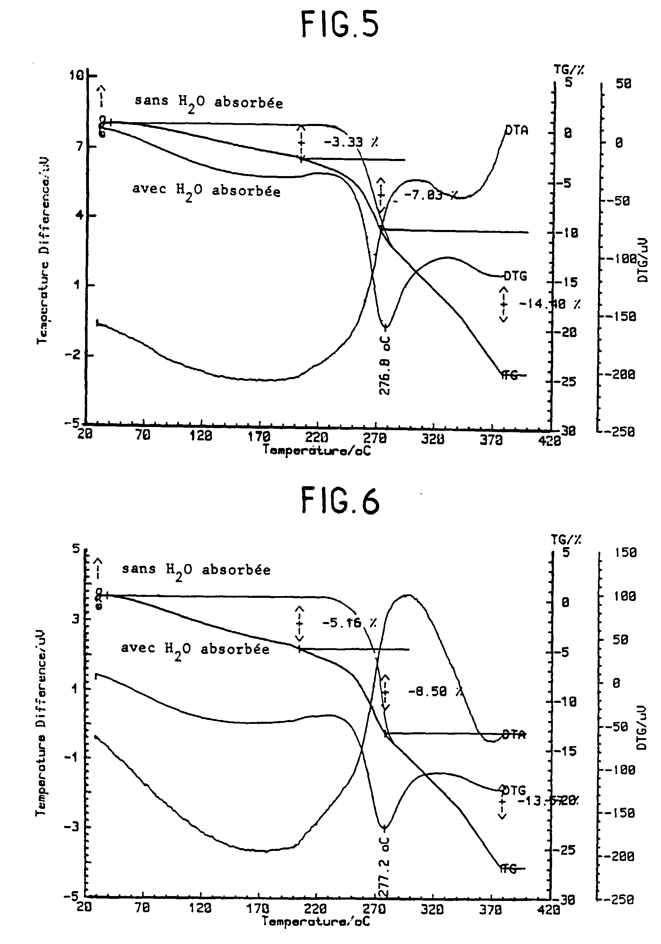

Afin de vérifier la stabilité thermique de ce matériau nous effectuons les tests traditionnels d'ATD-ATG. les résultats des tests sont représentés sur les figures 5 et 6 qui donnent le comportement thermique des matériaux totalement et partiellement deshydratés sous azote/oxygène (figure 5) d'une part, et d'autre part sous argon (figure 6), en opérant dans les conditions suivantes:

| | Figure 5 | Figure 6 |
|---|---|---|
| . échantillon : polyaniline | 99,90mg | 101,10mg |
| . référence: kaolin | 99,90mg | 99,80mg |
| . creuset | Al₂O₃ | Al₂O₃ |
| . TG | 125mg | 125mg |

On constate que ces matériaux ont une remarquable stabilité thermique puisqu'il est possible de les chauffer jusqu'aux environs de 250°C sans perte notable de masse.

### EXEMPLE 15

L'exemple 13 est répété mais, afin de faciliter la précipitation, on utilise 5ml d'acide sulfurique, 20ml d'eau et 40ml d'acide acétique. Cette fois, la normalité passe de 2,7N à 1,8N respectivement du début à la fin de la réaction, l'adjonction de la solution de peroxodisulfate d'ammonium ayant duré 7 heures; le rendement de cette réaction est de 27%.

### EXEMPLE 16 - Copolymérisation des monomères A3 et B

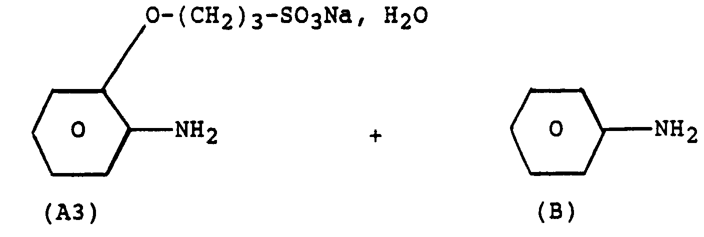

- A3 :: acide 3-(2-aminophénoxy)-1-propanesulfonique
- B :: aniline

La solution oxydante est versée goutte à goutte pendant une nuit dans un mélange thermostaté composé de 5g de monomère A3, 1,66g d'aniline et de 30ml d'acide sulfurique à 10% en volume. Le polymère est récupéré par centrifugation puis le culot est redispersé dans une solution aqueuse d'acide acétique à 10% en volume avant d'être centrifugé à nouveau. Cette opération qui consiste à laver le précipité peut être répétée plusieurs fois. Ce dernier est ensuite séché sous vide à 50°C durant 2 ou 3 jours. Le calcul du rendement est effectué par rapport à l'oxydant, suivant l'oxydant choisi, ce rendement est variable de 10% à 84%.

## Revendications

1. Polyaniline conductrice auto-dopée, caractérisée en ce que les noyaux benzéniques et/ou quinoniques de ladite polyaniline portent des greffons fonctionnalisés, ou substituants, correspondant à la formule:
-A-Z
dans laquelle:
A est un radical hydrocarboné de 2 à 8 atomes de carbone interrompu par au moins un hétéro-atome,
Z est un groupe fonctionnel choisi parmi les résidus d'un acide fort ou de l'un de ses sels.

2. Polyaniline selon la revendication 1, dans laquelle ledit hétéro-atome est choisi parmi O et S.

3. Polyaniline selon l'une des revendications 1 et 2, dans laquelle ledit groupe -A- est un résidu choisi parmi les résidus ayant une fonction éther ou polyéther, et ceux ayant une fonction ester.

4. Polyaniline selon la revendication 3, dans laquelle ledit résidu ayant une fonction éther ou polyéther est choisi parmi
-0-(CH₂)ₙ- et -(CH₂0)ₙ- avec n supérieur ou égal à 2,
-CH₂-0-(CH₂)ₙ- et -(CH₂-CH₂0)ₙ- avec n supérieur ou égal à 1.

5. Polyaniline selon la revendication 3, dans laquelle ledit résidu ayant une fonction ester est choisi parmi -C00-(CH₂)ₙ- et -CH=CH-C00-(CH₂)ₙ- avec n supérieur ou égal à 1.

6. Polyaniline selon la revendication 1, dans laquelle ledit groupe -A- comprend un atome d'oxygène dans sa chaîne et correspond à la formule:
-(CH₂)ᵣ-O-(CH₂)ₛ-
dans laquelle:
r est un entier valant 0 ou 1, et
s est un entier valant 3 ou 4.

7. Polyaniline selon l'une des revendications précédentes, dans laquelle ledit groupe -Z est un groupe fonctionnel choisi parmi les résidus d'acide sulfonique, phosphonique et phosphorique, et de leurs sels.

8. Polyaniline selon l'une des revendications précédentes, dans laquelle ladite polyaniline est sous une forme demi-oxydée à 75% de noyaux benzéniques et 25% de noyaux quinoniques, appelée polyéméraldine.

9. Polyaniline selon l'une des revendications précédentes, dans laquelle le nombre desdits greffons fonctionnalisés est égal à la moitié du nombre desdits noyaux benzéniques et/ou quinoniques.

10. Procédé de préparation d'une polyaniline selon l'une des revendications 1 à 9, comprenant une étape de copolymérisation en milieu réactionnel acide en présence d'un oxydant d'un premier monomère fonctionnalisé A de formule: 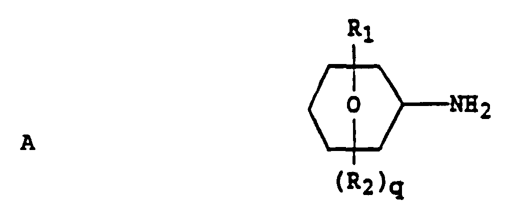 dans laquelle:
q vaut 0 ou 1,
R₁ et R₂, identiques ou différents, sont des groupes du type -A-Z dans lequel -A- est un radical hydrocarboné comportant de 2 à 8 atomes de carbone, interrompu par au moins un hétéro-atome, et -Z est un groupe fonctionnel choisi parmi les résidus d'un acide fort ou de l'un de ses sels,
et d'un deuxième monomère non fonctionnalisé B de formule: 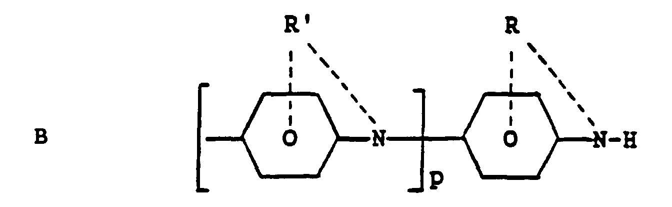 dans laquelle:
p vaut 0 ou 1,
R et R' identiques ou différents (la ligne en pointillés indiquant que R et/ou R' sont liés soit à l'atome d'azote, soit au noyau) sont choisis parmi:
. -H, -0H, un radical alkyle contenant de 1 à 12 atomes de carbone, un groupe -CH₂0H, -C₂H₄0H, -C00H, -0CH₃, et -0C₂H₅, lorsqu'ils sont rattachés au noyau,
. -H, -OH, un radical alkyle, un radical phényle, un groupe -CH₂0H, -C₂H₄0H, et -C00H, lorsqu'ils sont attachés à l'atome d'azote,
ladite copolymérisation comportant une phase d'induction durant laquelle l'oxydant est présent en faible quantité dans le milieu de copolymérisation, et une phase de production.

11. Procédé selon la revendication 10, dans lequel ledit monomère A est présent en totalité dans le milieu réactionnel au début de la réaction, et ledit monomère B et ledit oxydant sont ajoutés par incréments ou en continu sur la période des phases d'induction et de production, le rapport monomère B/oxydant étant sensiblement constant.

12. Procédé selon l'une des revendications 10 et 11, dans lequel ledit monomère B est également présent dans le milieu réactionnel de copolymérisation au cours de la phase d'induction.

13. Procédé selon la revendication 10, dans lequel lesdits monomères A et B sont présents en totalité dans le milieu réactionnel au début de la réaction, et ledit oxydant est ajouté par incréments ou en continu sur la période des phases d'induction et de production.

14. Procédé selon l'une des revendications 10 à 13, dans lequel le milieu réactionnel est soumis durant la phase d'induction à un chauffage, puis en ce qu'on laisse ensuite la température du milieu réactionnel évoluer de façon inertielle.

15. Procédé selon l'une des revendications 10 à 14, dans lequel le pH dudit milieu réactionnel est inférieur à 1.

16. Procédé selon l'une des revendications 10 à 15, dans lequel ledit milieu acide contient un acide choisi parmi l'acide chlorhydrique, l'acide sulfurique, l'acide phosphorique, leurs mélanges,et l'eutectique HF-NH₄F.

17. Procédé selon l'une des revendications 10 à 16, dans lequel ledit oxydant est choisi parmi KIO₃, H₂0₂, (NH₄)₂S₂0₈, et (NH₄)₂Cr₂0₇.

18. Procédé selon l'une des revendications 10 à 17, dans lequel ledit monomère B est l'aniline.

19. Procédé selon l'une des revendications 10 à 18, dans lequel ledit monomère A est de l'aniline monosubstituée ou disubstituée sur le noyau, en position ortho ou méta, de la formedu type:
(o)R₁₋aniline,
(m)R₁₋aniline,
(o)R₁,(o)R₂₋aniline,
(o)R₁,(m)R₂₋aniline,
(m)R₁,(o)R₂₋ₐₙᵢₗᵢₙₑ,
(m)R₁,(m)R₂₋aniline,
et leurs mélanges.

20. Procédé selon la revendication 17, dans lequel ledit monomère A est l'aniline disubstituée en ortho.

21. Procédé selon l'une des revendications 8 à 18, dans lequel ledit monomère A porte une fonction acide sulfonique.

22. Procédé selon l'une des revendications 8 à 19, dans lequel la préparation du monomère A consiste à partir d'un dérivé nitré aromatique portant au moins un groupement hydroxyle ou hydroxylé en position ortho ou méta:
. à réaliser le dérivé métallique de ce dérivé (alcoolate, phénolate),
. à faire réagir ce dernier sur une sultone en milieu anhydre, polaire et aprotique,
. à réduire la fonction "nitro" en fonction "amino",
. à récupérer le produit final sous forme de sel interne ou d'un dérivé salin quelconque en fonction de la commodité de séparation,
suivant le schéma réactionnel:

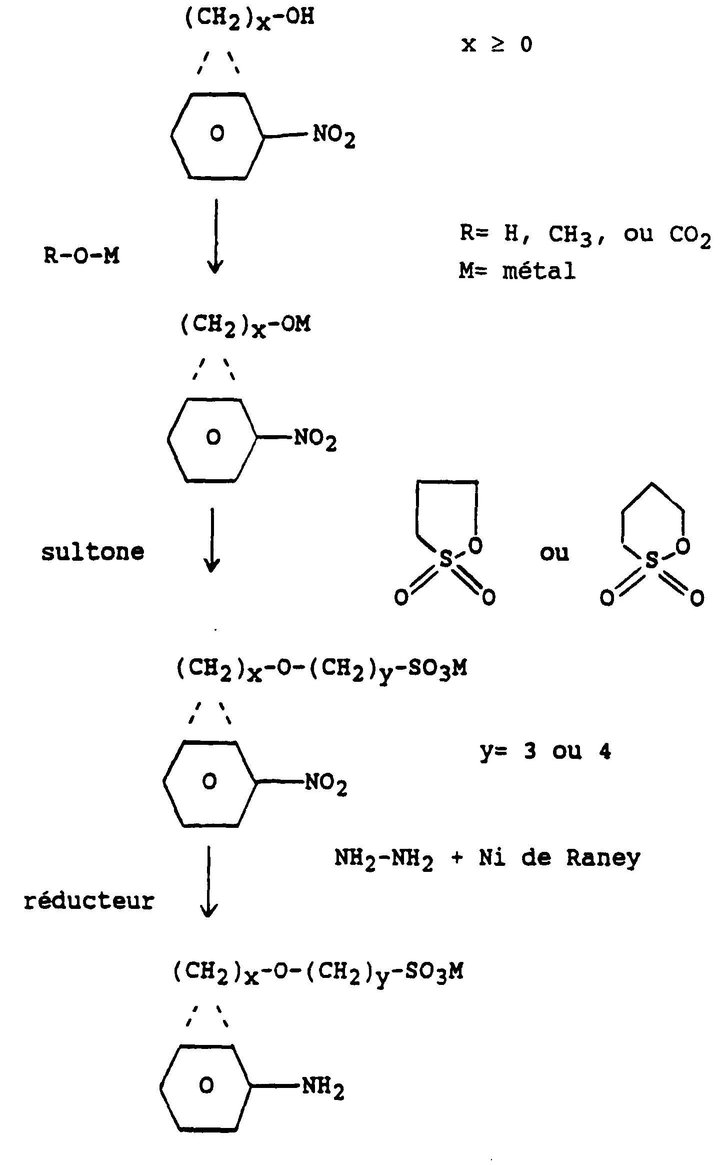

23. Procédé selon l'une des revendications 8 à 19, dans lequel la préparation des monomères de type A consiste à partir d'un dérivé aminé aromatique portant au moins un groupement hydroxyle ou hydroxylé en position ortho ou méta.
. à réaliser le dérivé métallique de ce dérivé (alcoolate, phénolate),
. à faire réagir ce dernier sur une sultone en milieu anhydre, polaire et aprotique,
. à récupérer le produit final sous forme de sel interne ou d'un dérivé salin quelconque en fonction de la commodité de séparation,
suivant le schéma réactionnel:

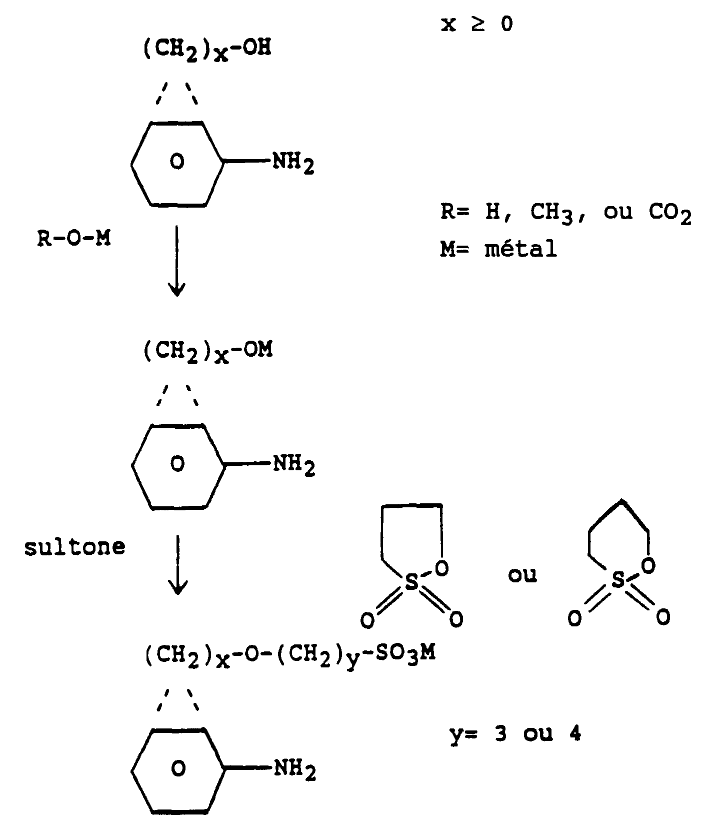

24. Polyaniline obtenue par le procédé selon l'une des revendications 10 à 23, dans laquelle on fait varier la conductivité de ladite polyaniline en faisant varier le rapport moléculaire monomère A/monomèreB.

25. Procédé de mise en oeuvre d'une polyaniline selon l'une des revendications 1 à 9, dans lequel ladite polyaniline est mise en solution dans un solvant.

26. Procédé selon la revendication 25, dans lequel ledit, solvant est choisi parmi une solution basique aqueuse et une solution basique organique.

27. Procédé selon l'une des revendications 25 et 26, dans lequel ladite polyaniline est mise en solution au moyen d'une base faible volatile.

28. Procédé selon l'une des revendications 25 à 27, dans lequel ladite solution est mise en oeuvre dans un procédé de revêtement par peinture, pistolage ou trempage.

29. Procédé selon l'une des revendications 25 à 28, appliqué à la réalisation de couches minces et de dépôts.

30. Utilisation d'une polyaniline selon l'une des revendications 1 à 9, dans le domaine électromagnétique.

## Patentansprüche

1. Leitendes, selbstdotiertes Polyanilin, dadurch gekennzeichnet, daß die Benzol- und/oder Chinonkerne der Polyanilins funktionalisierte Implantate oder Substituenten tragen, die der Formel
-A-Z
entsprechen, wobei
A ein kohlenwasserstoffhaltiges Radikal mit 2 bis 8 Kohlenstoffatomen unterbrochen durch mindestens ein Heteroatom ist,
und Z eine funktionale Gruppe ist, die aus den Residuen einer starken Säure oder einem ihrer Salze ausgewählt ist.

2. Polyanilin nach Anspruch 1, bei dem das Heteroatom ausgewählt wird aus O und S.

3. Polyanilin nach einem der Ansprüche 1 und 2, bei dem die Gruppe -A- ein Residuum ist, das aus den Residuen mit einer Äther- oder Polyäther-Funktion und den Residuen mit einer Esterfunktion ausgewählt wird.

4. Polyanilin nach Anspruch 3, bei dem das Residuum mit einer Äther- oder Polyätherfunktion ausgewählt wird aus -O-(CH₂)ₙ-, -(CH₂O)ₙ- mit n mindestens gleich 2 und aus -CH₂-O-(CH₂)ₙ-, -(CH₂-CH₂O)ₙ- mit n mindestens gleich 1.

5. Polyanilin nach Anspruch 3, bei dem das Residuum mit einer Esterfunktion ausgewählt wird aus-COO-(CH₂)ₙ- und -CH=CH-COO-(CH₂)ₙ-, mit n mindestens gleich 1.

6. Polyanilin nach Anspruch 1, bei dem die Gruppe -A- in ihrer Kette ein Sauerstoffatom enthält und der Formel entspricht:
-(CH₂)ᵣ-O-(CH₂)ₛ-
in der
r eine ganze Zahl 0 oder 1 ist und
s eine ganze Zahl 3 oder 4 ist.

7. Polyanilin nach einem der vorhergehenden Ansprüche, bei dem die Gruppe -Z eine funktionale Gruppe ist, die aus den Residuen von Sulfonsäure, Phosphonsäure und Phosphorsäure und ihren Salzen ausgewählt wird.

8. Polyanilin nach einem der vorhergehenden Ansprüche, bei dem das Polyanilin in einer halboxidierten Form vorliegt mit 75% Benzolkernen und 25% Chinonkernen, die Polyemeraldin genannt wird.

9. Polyanilin nach einem der vorhergehenden Ansprüche, bei dem die Zahl der funktionalisierten Implantate gleich der Hälfte der Zahl von Benzol- und/ oder Chinonkernen ist.

10. Verfahren zur Herstellung eines Polyanilins nach einem der Ansprüche 1 bis 9, das in saurem Reaktionsmilieu und in Gegenwart eines Oxidationsmittels einen Schritt der Copolymerisation eines ersten funktionalisierten Monomers A der Formel 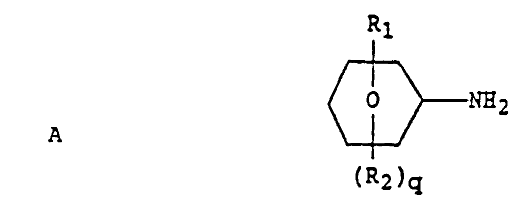 in der
q gleich 0 oder 1 ist
R₁ und R₂ identisch oder unterschiedlich sind und Gruppen vom Typ -A-Z bilden, wobei -A- ein kohlenwasserstoffhaltiges Radikal mit 2 bis 8 Kohlenstoffatomen, unterbrochen durch mindestens ein Heteroatom, und -Z eine funktionale Gruppe ist, die aus den Residuen einer starken Säure oder eines ihrer Salze gewählt wird,
und eines zweiten nicht-funktionalisierten Monomers B der folgenden Formel enthält 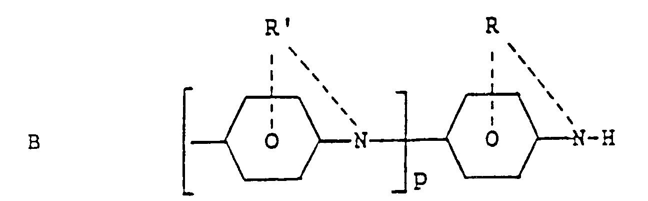 in der
p gleich 0 oder 1 ist,
R und R' identisch oder unterschiedlich sind (die gestrichelte Linie gibt an, daß R und/oder R' entweder mit dem Stickstoffatom oder mit dem Kern verbunden sind) und ausgewählt werden aus:
. -H, -OH, einem Alkylradikal mit 1 bis 12 Kohlenstoffatomen, einer Gruppe -CH₂OH, -C₂H₄OH, -COOH, -OCH₃ und -OC₂H₅, wenn sie mit dem Kern verbunden sind,
. -H, -OH, einem Alkylradikal, einem Phenylradikal, einer Gruppe -CH₂OH, -C₂H₄OH, -COOH, wenn sie mit dem Stickstoffatom verbunden sind,
wobei die Copolymerisation eine Induktionsphase, während der das Oxidationsmittel in geringer Menge im Copolymerisationsmilieu vorhanden ist, und eine Produktionsphase enthält.

11. Verfahren nach Anspruch 10, bei dem das Monomer A vollständig im Reaktionsmilieu zu Beginn der Reaktion vorhanden ist und das Monomer B und das Oxidationsmittel schrittweise oder kontinuierlich über die Induktions- und die Produktionsphase hinzugefügt werden, wobei das Verhältnis zwischen Monomer B und Oxidationsmittel im wesentlichen konstant ist.

12. Verfahren nach einem der Ansprüche 10 und 11, bei dem das Monomer B auch im Copolymerisations-Reaktionsmilieu während der Induktionsphase vorhanden ist.

13. Verfahren nach Anspruch 10, bei dem die Monomere A und B vollständig im Reaktionsmilieu zu Beginn der Reaktion vorhanden sind und das Oxidationsmittel in Schritten oder kontinuierlich über die Periode der Induktions- und der Produktionsphase hinzugefügt werden.

14. Verfahren nach einem der Ansprüche 10 bis 13, bei dem das Reaktionsmilieu während der Induktionsphase einer Erwärmung unterworfen wird und man dann die Temperatur des Reaktionsmilieus sich inert entwickeln läßt.

15. Verfahren nach einem der Ansprüche 10 bis 14, bei dem der pH-Wert des Reaktionsmilieus unter 1 liegt.

16. Verfahren nach einem der Ansprüche 10 bis 15, bei dem das Säuremilieu eine Säure enthält, die aus Salzsäure, Schwefelsäure, Phosphorsäure und ihren Mischungen sowie dem Eutektikum HF-NH₄F ausgewählt wird.

17. Verfahren nach einem der Ansprüche 10 bis 16, bei dem das Oxidationsmittel ausgewählt wird aus KIO₃, H₂O₂, (NH₄)₂S₂O₈ und (NH₄)₂Cr₂O₇.

18. Verfahren nach einem der Ansprüche 10 bis 17, bei dem das Monomer B Anilin ist.

19. Verfahren nach einem der Ansprüche 1 bis 18, bei dem das Monomer A ein auf den Kern in der ortho- oder meta-Position mono- oder disubstituiertes Anilin der folgenden Form ist
(o)R₁-Anilin,
(m)R₁-Anilin,
(o)R₁,(o)R₂-Anilin
(o)R₁,(m)R₂-Anilin,
m(R₁),(o)R₂-Anilin,
(m)R₁,(m)R₂-Anilin
und deren Mischungen.

20. Verfahren nach Anspruch 17, bei dem das Monomer A in ortho-Position disubstituiertes Anilin ist.

21. Verfahren nach einem der Ansprüche 8 bis 18, bei dem das Monomer A eine Sulfonsäure-Funktion trägt.

22. Verfahren nach einem der Ansprüche 8 bis 19, bei dem die Herstellung des Monomers des Typs A darin besteht, ausgehend von einem aromatischen nitrierten Derivat, das mindestens eine Hydroxyl-Gruppierung oder eine hydroxylierte Gruppierung in der ortho- oder meta-Position trägt,
. das metallische Derivat dieses Derivats herzustellen (Alkoholat, Phenolat),
. dieses letztere mit einem Sulton in einem wasserfreien, polaren und aprotischen Milieu reagieren zu lassen,
. die "nitro"-Funktion in eine "amino"-Funktion zu reduzieren,
. das Endprodukt in Form eines inneren Salzes oder eines beliebigen Salzderivats in Abhängigkeit von der Trennbequemlichkeit zu gewinnen gemäß dem Reaktionsschema:

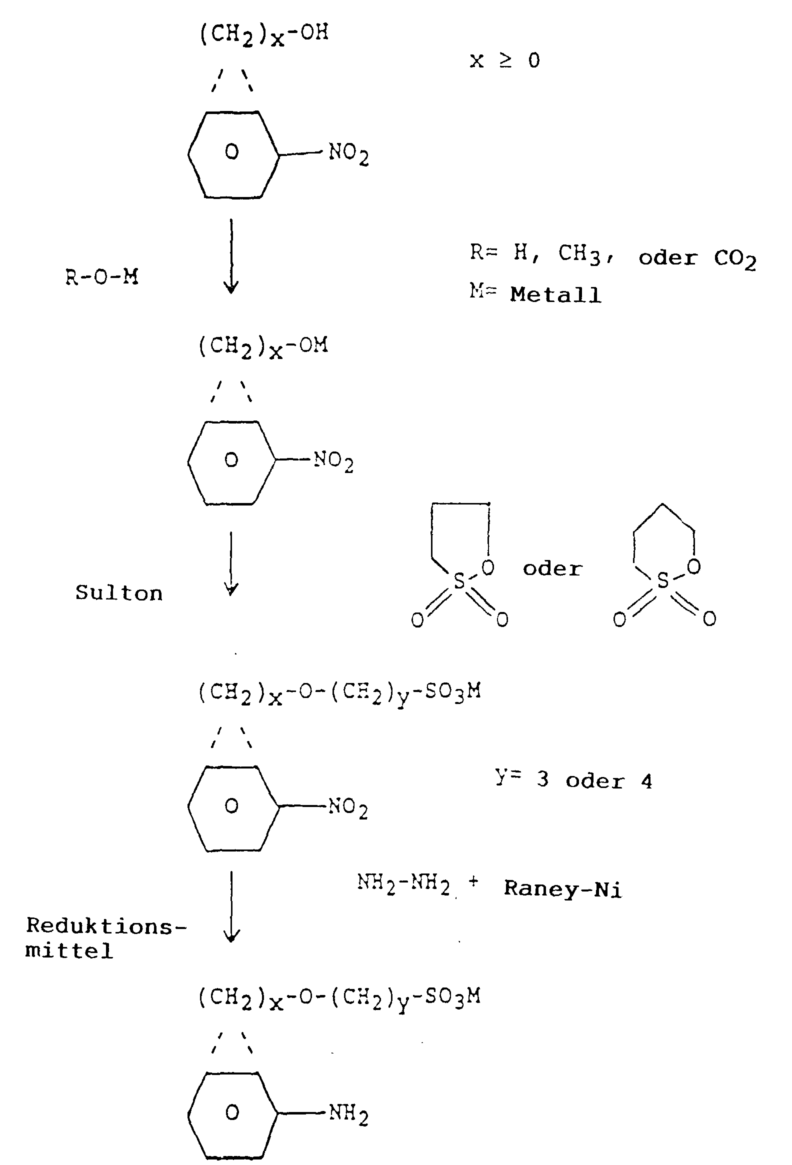

23. Verfahren nach einem der Ansprüche 8 bis 19, bei die Herstellung der Monomere des Typs A darin besteht, ausgehend von einem aromatischen Aminoderivat, das mindestens eine Hydroxylgruppierung oder eine hydroxylierte Gruppierung in der ortho- oder meta-Position trägt,
- das Metallderivat dieses Derivats herzustellen (Alkoholat, Phenolat),
. dieses letztere mit einem Sulton in wasserfreiem, polarem und aprotischem Milieu reagieren zu lassen,
. das Endprodukt in Form eines inneren Salzes oder eines beliebigen Salzderivats in Abhängigkeit von der Trennbequemlichkeit zu gewinnen gemäß dem folgenden Reaktionsschema:

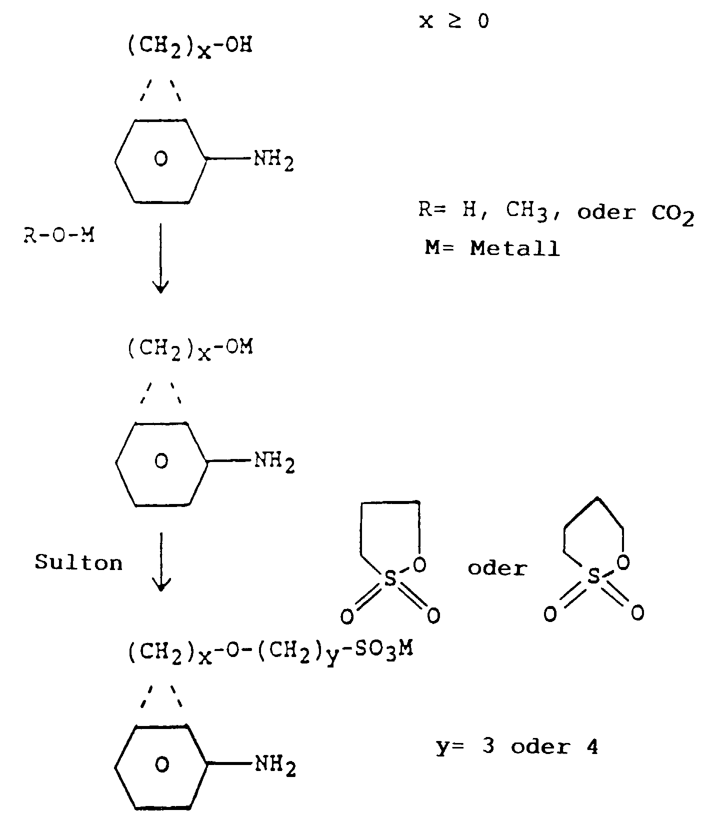

24. Gemäß dem Verfahren nach einem der Ansprüche 10 bis 23 erhaltenes Polyanilin, bei dem man die Leitfähigkeit des Polyanilins dadurch variiert, daß man das molekulare Verhältnis Monomer A/Monomer B variiert.

25. Verfahren zur Anwendung eines Polyanilins gemäß einem der Ansprüche 1 bis 9, bei dem das Polyanilin in einem Lösungsmittel gelöst wird.

26. Verfahren nach Anspruch 25, bei dem das Lösungsmittel ausgewählt wird aus einer wässrigen basischen Lösung und eine organischen basischen Lösung.

27. Verfahren nach einem der Ansprüche 25 und 26, bei dem das Polyanilin mittels einer schwachen flüchtigen Base in Lösung gebracht wird.

28. Verfahren nach einem der Ansprüche 25 bis 27, bei dem die Lösung in einem Beschichtungsverfahren durch Anstreichen, Aufspritzen oder Eintauchen verwendet wird.

29. Verfahren nach einem der Ansprüche 25 bis 28, das zur Herstellung dünner Schichten und Beschichtungen verwendet wird.

30. Verwendung eines Polyanilins gemäß einem der Ansprüche 1 bis 9 im elektromagnetischen Bereich.

## Claims

1. Self-doped conductive polyaniline, characterized in that the benzene and/or quinone rings of said polyaniline carry functionalized grafted chains or substituents corresponding to the formula:
-A-Z
in which:
A is a hydrocarbon radical having 2 to 8 atoms of carbon interrupted by at least one hetero atom; and
Z is a functional group selected from residues of a strong acid or a salt thereof.

2. Polyaniline according to claim 1, in which said hetero atom is selected from O and S.

3. Polyaniline according to claim 1 or 2, in which said -A- group is a residue selected from residues having an ether or a polyether function, and those having an ester function.

4. Polyaniline according to claim 3, in which said residue having an ether or a polyether function is selected from:
-O-(CH₂)ₙ- and -(CH₂O)ₙ- where n is greater than or equal to 2;
-CH₂-O-(CH₂)ₙ- and -(CH₂-CH₂O)ₙ- with n greater than or equal to 1.

5. Polyaniline according to claim 3, in which said residue having an ester function is selected from:
-COO-(CH₂)ₙ- and -CH=CH-COO-(CH₂)ₙ- with n greater than or equal to 1.

6. Polyaniline according to claim 1, in which said -A- group includes an oxygen atom in its chain and corresponds to the formula:
-(CH₂)ᵣ-O-(CH₂)ₛ-
in which:
r is an integer equal to 0 or 1; and
s is an integer equal to 3 or 4.

7. Polyaniline according to any preceding claim, in which said -Z-group is a functional group selected from residues of sulfonic, phosphonic, and phosphoric acids and salts thereof.

8. Polyaniline according to any preceding claim, in which said polyaniline is in a semi-oxidized form corresponding to 75% benzene rings and 25% quinone rings, called polyemeraldine.

9. Polyaniline according to any preceding claim, in which the number of said functionalized grafted chains is equal to one-half the number of said benzene and/or said quinone rings.

10. A method of preparing polyaniline according to any one of claims 1 to 9, including a step of copolymerizing a functionalized first monomer A in an acid reaction medium and in the presence of an oxidizer, and having the following formula: 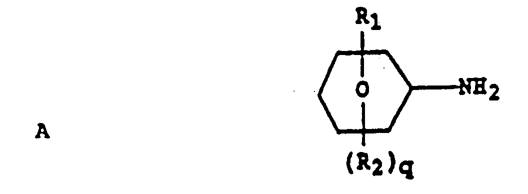 in which:
q is 0 or 1;
R₁ and R₂ are equal or different and are groups of the -A-Z type in which -A- is a hydrocarbon containing a radical having 2 to 8 atoms of carbon and interrupted by at least 1 hetero atom, and -Z is a functional group selected from residues of a strong acid or a salt thereof,
and a non-functionalized second monomer B having the following formula: 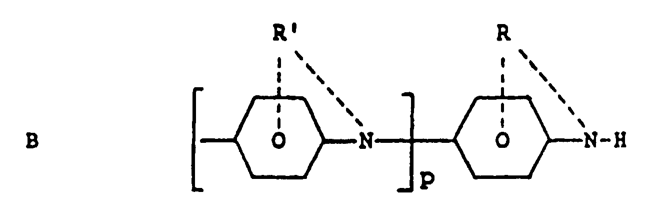 in which:
p is 0 or 1;
R and R' which are identical or different (with the dashed lines indicating that R and/or R' are bonded either to the nitrogen atom or the ring) are selected from:
-H, -OH, an alkyl radical containing 1 to 12 atoms of carbon, or one of the following groups: -CH₂OH, -C₂H₄OH, -COOH, -OCH₃, and -OC₂H₅ when they are bonded to the ring;
-H, -OH, an alkyl radical, a phenyl radical, or one of the following groups: -CH₂OH, -C₂H₄OH, and -COOH when they are attached to the nitrogen atom,
said copolymerization including an induction stage during which the oxidizer is present in small quantity in the copolymerization medium, and a production stage.

11. A method according to claim 10, in which said monomer A is fully present in the reaction medium at the beginning of the reaction, and said monomer B and said oxidizer are added in increments or continuously during the period of the induction stage and of the production stage, the ratio of monomer B to oxidizer being substantially constant.

12. A method according to claim 10 or 11, in which said monomer B is also present in the copolymerization reaction medium during the induction stage.

13. A method according to claim 10, in which said monomers A and B are fully present in the reaction medium at the beginning of the reaction, and said oxidizer is added in increments continuously over the period of the induction stage and of the production stage.

14. A method according to any one of claims 10 to 13, in which the reaction medium is subjected during the induction stage to heating, after which the temperature of the reaction medium is allowed to change under its own inertia.

15. A method according to any one of claims 10 to 14, in which the pH of said reaction medium is less than 1.

16. A method according to any one of claims 10 to 15, in which said acid medium contains an acid selected from hydrochloric acid, sulfuric acid, phosphoric acid, mixtures thereof, and eutectic HF-NH₄F.

17. A method according to any one of claims 10 to 16, in which said oxidizer is selected from KIO₃, H₂O₂, (NH₄)₂S₂O₈, and (NH₄)₂Cr₂O₇.

18. A method according to any one of claims 10 to 17, in which said monomer B is aniline.

19. A method according to any one of claims 10 to 18, in which said monomer A is aniline mono-substituted or di-substituted on the ring in the ortho position or in the meta position, of the form:
(o)R₁-aniline,
(m)R₁-aniline,
(o)R₁,(o)R₂-aniline,
(o)R₁,(m)R₂-aniline,
(m)R₁,(o)R₂-aniline,
(m)R₁,(m)R₂-aniline,
and mixtures thereof.

20. A method according to claim 17 in which said monomer A is ortho disubstituted aniline.

21. A method according to any one of claims 8 to 18, in which said monomer A carries a sulfonic acid function.

22. A method according to any one of claims 8 to 19, in which preparing monomer A consists, starting from an aromatic nitrated derivative carrying at least one hydroxyl or hydroxylated group in the ortho position or the meta position:
in obtaining the metallic derivative of said derivative (alcoholate, phenolate);
reacting it with a sultone in a medium that is anhydrous, polar, and aprotic;
reducing the nitro function into an amino function; and
recovering the final product in the form of an internal salt or any salt derivative as a function of convenience in separation; following the reaction scheme:

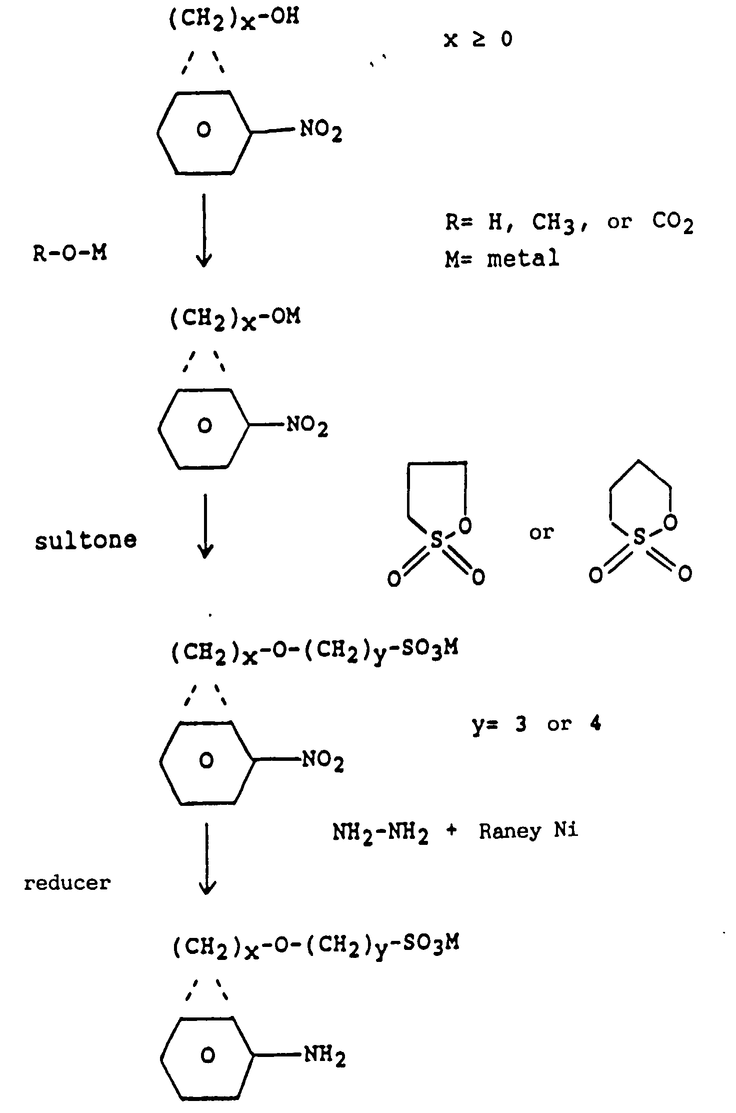

23. A method according to any one of claims 8 to 19, in which preparing type A monomers consists in starting from an aromatic amine-containing derivative carrying at least one hydroxyl or hydroxylated group in the ortho position or in the meta position:
in obtaining the metallic derivative of said derivative (alcoholate, phenolate);
in reacting it with a sultone in a medium that is anhydrous, polar, and aprotic; and
in recovering the final product in the form of an internal salt or of any salt derivative as a function of separation convenience; following the reaction scheme:

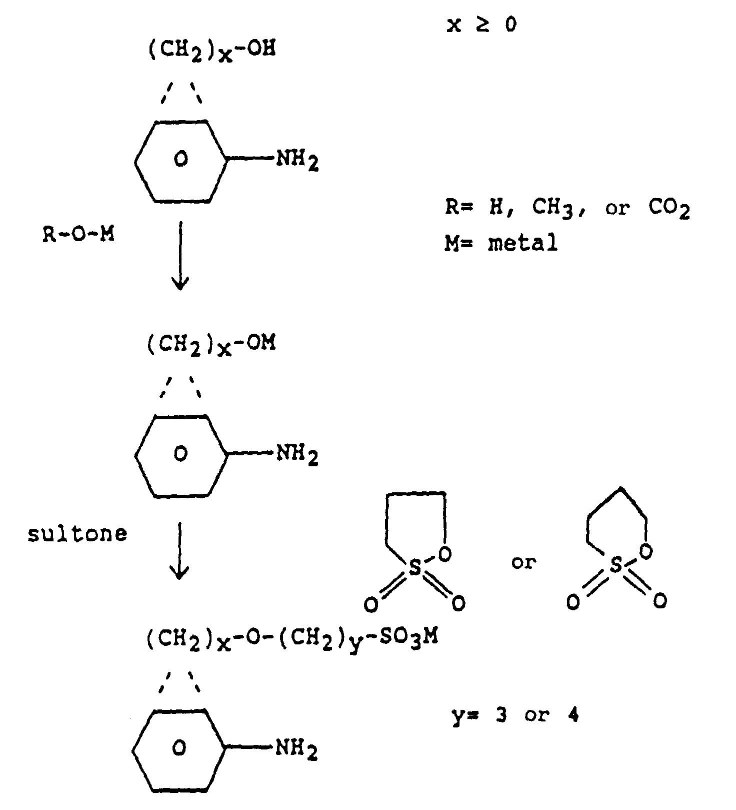

24. Polyaniline obtained by the method according to any one of claims 10 to 23, in which the conductivity of said polyaniline can be varied by varying the molecular ratio of monomer A to monomer B.

25. A method of implementing a polyaniline according to any one of claims 1 to 9, in which said polyaniline is put into solution in a solvent.

26. A method according to claim 25, in which said solvent is selected from an aqueous basic solution and an organic basic solution.

27. A method according to claim 25 or 26, in which said polyaniline is put into solution by means of a volatile weak base.

28. A method according to any one of claims 25 to 27, in which said solution is implemented in a coating method by painting, spraying, or soaking.

29. A method according to any one of claims 25 to 28, applied to making thin layers and deposits.

30. Using polyaniline according to any one of claims 1 to 9, in the field of electromagnetics.
